(19) Europäisches Patentamt
European Patent Office
Office européen des brevets

(11) EP 0 612 705 B1

(12) EUROPEAN PATENT SPECIFICATION

(45) Date of publication and mention of the grant of the patent: **10.07.1996 Bulletin 1996/28**

(51) Int. Cl.[6]: **C07C 11/00**, C07C 7/20, C10G 70/06

(21) Application number: **94200425.0**

(22) Date of filing: **21.02.1994**

(54) **Method for preventing fouling in unsaturated hydrocarbon**

Verfahren zur Vermeidung von Verschmutzung in ungesättigten Kohlenwasserstoffen

Procédé pour éviter l'encrassement dans des hydrocarbures insaturés

(84) Designated Contracting States:
**BE DE FR GB IT NL**

(30) Priority: **25.02.1993 BE 9300176**

(43) Date of publication of application:
**31.08.1994 Bulletin 1994/35**

(73) Proprietor: **CHARON HOLDING, s.a.**
**1724 Luxembourg (LU)**

(72) Inventor: **Vercammen, Fernand S.J.**
**B-2460 Kasterlee (BE)**

(74) Representative: **Donné, Eddy**
**Bureau M.F.J. Bockstael nv**
**Arenbergstraat 13**
**2000 Antwerpen (BE)**

(56) References cited:
**US-A- 3 527 821**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 612 705 B1

## Description

The invention concerns a method for preventing fouling due to condensation reactions of one or more carbonyls present or formed in unsaturated hydrocarbons.

Such fouling usually takes place as a result of free radical polymerization which is chemically and/or thermally initiated by contaminants such as oxygen or metal ions, or condensation reactions of alpha, beta unsaturated carbonyl compounds and the like (Aldol condensation, Cannizzaro reactions, Knoevenagel condensation, Perkin reactions, Diels-Alder addition reactions).

The recovering of alkenes from gas or liquids, the transformation of alkene or acetyl compounds by means of distillation, the catalytic hydrogenation, dehydrogenation or dehydrochlorogenation, the pyrolytic dehydrogenation or dehydrochlorogenation, acylation, alkylation, chemical and physical adsorption and absorption, compression, extraction, etc. are hindered by such fouling. Not only during these processes, but also during the storage and the transport of the unsaturated hydrocarbons can fouling occur on the equipment used.

In particular during the extraction and purification of 1,3 butadiene by solvents such as furfural, acetonitrile, dimethylacetamide, N-methylpyrrolidone or dimethylformamide, depositions of polymeric elements hinder during the extraction, the finishing operation, the gas recovering and storage.

Also during the production of ethylbenzene and cumene with the so-called wet alkylation (Friedel-Crafts catalysts), unwanted by-products and organic polymers arise in the reaction itself, the catalytic neutralisation and product recovering.

Fouling also takes place in the units for the absorption/adsorption and chemical conversion of organic and inorganic sulphur compounds during the treatment of hydrocarbons, in particular during the removal of hydrogen sulphide, thiophenes, mercaptans and other organic and inorganic sulphur compounds from natural gas and oil products or the processing thereof.

The invention aims to provide a method according to which the formation of these unwanted fouling is prevented in a simple manner.

To this aim, an additive formed by a solution of at least one hydride in a solvent is added to the unsaturated hydrocarbon.

US-A-3 527 821 discloses the incorporation of an effective amount of discrete particles of finely divided sodium borohydride in olefinic hydrocarbon. This is however not to prevent condensation reactions of carbonyls but to destruct hydroperoxid contaminants and thus to stabilize the olefinic hydrocarbon material.

The solvent may be an alkali hydroxide solution.

The concentration of the hydride in the solvent practically amounts to 1 to 25 % by weight.

Suited hydrides are among others boro or aluminium hydrides and especially alkali borohydrides or alkali aluminium hydrides.

The amount of additive depends from one case to another but practically amounts to between 0.0010 and 10 % by weight, dosed on the amount of hydrocarbon.

According to a preferred embodiment of the invention, an amount of promoter is also added.

This promoter can be either added directly to the hydrocarbons or it can be mixed with the hydride in the additive.

Suited promoters are $Pd(NO_3)_2$, $AlCl_3$, $CoCl_2$, $NiCl_2$, thiols, acetic acid, $TiCl_4$, diethyleneglycoldimethylether, glycolic acid, gallic acid, thiourea or mixtures thereof.

The amount of promoter practically amounts to between 0.0010 and 10 % by weight, dosed on the amount of hydrocarbon.

Other particularities and advantages of the invention will become clear from the following description of a method for preventing fouling in unsaturated hydrocarbon. This description is given as an example only and does not limit the invention in any way.

In order to prevent fouling or deposition due to the presence or the formation of one or more carbonyls in unsaturated hydrocarbons, a small amount of additive formed by a solution of a hydride or a mixture of hydrides in a solvent, is added according to the invention.

Particularly, this additive is added just before or during a washing or extraction process to which the hydrocarbon is submitted.

Suited hydrides are especially boro and aluminium hydrides, in particular alkali borohydrides and alkali aluminium hydrides, which are preferably solved in an alkali hydroxide solution.

Useful hydrides are for example hydrides from the group: $LiBH_4$, $LiBH(C_2H_5)_3$, $NaBH_4$, $NaBH_3CN$, $KBH_4$, $LiAlH_4$, $LiAlH(OC(CH_3)_3)_3$, $NaAlH_4$, $NaAlH_2(OC_2H_4OCH_3)_2$, $NaAlH_2(C_2H_5)_2$, or mixtures thereof.

The hydrogen atoms of the hydride react with the unsaturated bond of the hydrocarbon so as to form an alcoholate which is disintegrated by means of hydrolysis. The reactions are as follows:

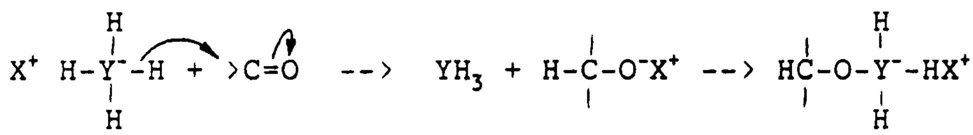

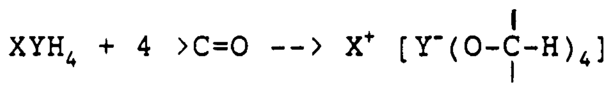

$$X^+ [Y^-(O\text{-}C\text{-}H<)_4] + 2H_2O \text{ --> } 4 >CH\text{-}OH + XYO_2$$

whereby

X = Na or Li and

y = B or Al

The concentration of the hydride in the solution amounts to between 1 and 25 % by weight.

Practically, also 0.001 to 10 % by weight of an organic or inorganic promoter, calculated on the amount of hydrocarbon, is added either directly to the hydrocarbon or mixed with the hydride, thus preferably in the solution of the hydride.

Suited promoters are substances from the group: $Pd(NO_3)_2$, $AlCl_3$, $CoCl_2$, $NiCl_2$, thiols, acetic acid, $TiCl_4$, diethyleneglycoldimethylether, glycol acid, gallic acid, thiourea or mixtures thereof.

The amount of added additive, whether or not with promoter, amounts to 0.001 % by weight to 10 % by weight of the amount of hydrocarbon, for example the process stream.

Said additive can be usefully added to restrict the deposition in unsaturated hydrocarbons during the transport or production or treatment thereof, and among others during the recovering of alkenes from gas or liquid, the conversion of olefinic or acetylic compounds by means of distillation, the catalytic hydrogenation, dehydrogenation or dehydrochlorogenation, the pyrolytic dehydrochlorogenation, acylation, alkylation, chemical and physical adsorption and absorption, compression, extraction, and during the production of ethylbenzene and cumene with the so-called wet alkylation (Friedel-Crafts catalysts), the extraction and purification of 1,3 butadiene with the help of solvents such as furfural, acetonitrile, dimethylacetamide, N-methylpyrrolidone or dimethylformamide, etc.

A particular application of the invention concerns the removal of $H_2S$ and $CO_2$ from the gases resulting of the thermal cracking of liquid or gaseous hydrocarbons, whereby use is made of alkanolamines (MEA, DEA, MDEA, ADIP), sterically hindered amines, alkazide and alkali solutions.

More in particular the above-mentioned additive is added to the NaOH washing tower which treats the cooled and compressed cracking gases of an ethylene installation. In this tower, a solution of NaOH is pumped round in water in order to remove $H_2S$ and $CO_2$ from these cracking gases.

These gases contain measurable amounts of carbonyl such as aldehydes, ketones, acetylenes, carboxylated esters and such like. Aldehydes and ketones, which may be formed by means of direct oxidation of ethylene, hydrocatalysation with $PdCl_2$ or hydroformylation of alkenes, appear to be precursors for the formation of a resinous deposition called "Red Tide" on parts of the installation, as a result of which the effectiveness of the purification and the efficiency decrease. In the presence of alkali or alkaline-earth hydroxides, these aldehydes first form acetaldol (3-hydroxybutanal) which is transformed at a higher temperature into crotonaldehyde which polymerizes with aldehydes and ketones. This resin formation is stimulated by traces of oxygen, the presence of iron salts of crotonic acid, reaction products of methanol or ethanol in the presence of NaOH to 3-alkoxybutyraldehydes, polyester-forming organic acids, reaction products of ammonia solution and acetaldehyde, etc.

Ketones, which are formed in an analogous manner as aldehydes, form diacetone alcohol or benzyl acetone. Other components of the deposition may be formed by reactions of aldehydes or ketones with ammonia solution, hydrazine, diethylhydroxylamines, semicarbazide and primary amines.

Another special application of the above-mentioned additive is with the solvent extraction of mixed 1,3-butadiene streams, whereby this extraction contains a pre-wash for the removal of oxygen, a treatment with a aqueous solution of $NaNO_2$ for the removal of carbonyls, a pre-fractionation for the precipitation of heavy components, an actual solvent extraction in two steps followed by the recovering of the solvent and a fractionation in two steps for the final purification.

There is never a complete removal of oxygen and carbonyl compounds, and both in the extraction part and the finishing part depositions may arise, especially due to the catalytic action of iron. The accumulation of vinyl acetylenes and the getting out of hand of the temperature in the finishing part have for a result that especially in the upper part of the fractionating column and in the upper gas condenser, polymer deposits may lead to dangerous pressure build-ups.

In order to avoid this, $NaNO_2$, p-tert-butylcathechol or amines such as diethylhydroxylamines can be added, as is already known. $NaNO_2$ is very toxic, however; it gives cause to the formation of toxic nitrosamines and may only be used in the liquid phases in the extraction and finishing parts. The amines reduce the selectivity of the solvent and may form nitrosamines or even deposits as a result of their reaction with the solvent, whereas the removal thereof from the butadiene is relatively expensive.

The addition of the additive according to the invention has the same or an even better result without the abovementioned disadvantages.

The invention can also be applied in units for the absorption/adsorption and the chemical conversion of organic and inorganic sulphur compounds in the treatment of hydrocarbons.

A special application of the invention regards the removal of hydrogen sulphide, thiophenes, mercaptans and other organic or inorganic sulphur compounds from natural gas and oil products or the processing thereof, as with the absorption and adsorption processes with alkanolamines, sterically hindered amines, glycolethers, active carbon and such like or with the conversion of mercaptanes to disulphides in catalytic processes with chemical and physical absorption (Merox).

The invention will be further illustrated by means of two practical examples.

Example 1:

A solution of caustic soda, saturated with oxygen and with the following composition, i.e. a solution with an analogous composition as in the caustic soda washing tower of an ethylene installation, was heated to 50°C and pumped round for 5 days in a tower on reduced scale.

| | |
|---|---|
| NaOH | 12.00 % by weight |
| $Na_2S$ | 0.32 % by weight |
| $NaHCO_3$ | 0.15 % by weight |
| Acetaldehyde | 0.02 % by weight |
| Vinylacetate | 0.005 % by weight |
| Cumene hydroperoxide | 0.005 % by weight |
| Acetic acid | 0.002 % by weight |
| Water | 86.498 % by weight |

After five days, the solution was treated with acid and extracted with dichloromethane. The extract was evaporated in vacuum and the residue was weighed and examined for resins.

Before the test was started, the solution was treated with different amounts of an added additive consisting of a solution of 12 % by weight $NaBH_4$ formulated with 0.1 % by weight diethyleneglycoldimethylether as a promoter in NaOH. In comparison, two amounts of the solution were treated without any additive. The results are given in the following table:

| Amount of additive in p.p.m. on the total amount of solution | residue in the extract in p.p.m. | resin in p.p.m. |
|---|---|---|
| 0 | 1052 | 876 |
| 0 | 1064 | 880 |
| 200 | 339 | 152 |
| 400 | 208 | 21 |
| 800 | 204 | 18 |
| 1600 | 212 | 19 |

The solutions containing no additives darkened after only one hour, whereas the solutions containing an additive preserved their pale yellow colour. As from 400 p.p.m. of additive, the amount of resin was 40 times smaller.

Example 2

For the cracking gas which was supplied to the washing tower with NaOH of an industrial ethylene installation and whose composition is given hereafter, 400 p.p.m. calculated on the total gas stream was added of an added additive consisting of a solution containing 12 % by weight $NaBH_4$ and 0.1 % by weight diethylene glycoldimethylether as a promoter in NaOH.

| | |
|---|---|
| $H_2S$ | 0.20 % by weight |
| $CO_2$ | 0.12 % by weight |
| Dienes | 5.85 % by weight |
| Aldehydes | 160 p.p.m. |
| Carbonyls | 0.11 % by weight |
| Peroxides | 35 p.p.m. |
| Ammonia solution | 2.5 p.p.m. |

The additive was added after the washing tower was cleaned, and after 3 months, the gas flow in the washing tower was still 100% without any sign of deposition, foaming or loss of pressure. The spent caustic had preserved its original pale yellow colour. Before, when no additive was used, the gas flow had dropped to 85% over a same period of time, whereas the pH of the washing water had risen from 8.5 to 11, whereas the pH of the washing water kept its value of 8.5 during the test. The typical red colour of the spent caustic did not appear during the test. By adding the additive, the total amount of extractable organic matter in the spent caustic dropped from 0.13 to 0.004 %, so that the used washing liquid could still be used in the paper industry.

So, the additive can clearly prevent the condensation reactions of the carbonyl compounds during the washing with caustic soda. As a result, the maximum gas flow can be kept at the same level for a long time, and the caustic soda is prevented from being swept along from the washing tower, which may for example affect the aluminium packings. The spent caustic must not even be recleaned with petrol, which is quite economic. Since the used NaOH can be recycled for example in the paper industry, this reduces the waste problem.

The addition of the above-described additive can keep the reaction of alpha, beta unsaturated compositions under control and can prevent the catalytically initiated auto-polymerization of alkene compositions. In particular, this additive can prevent the formation of depositions in the alkaline washing towers in the ethylene production or in the installations for the extraction of 1,3 butadiene.

The invention is by no means limited to the above-described embodiments; on the contrary, such a method for preventing deposition due to contaminants in unsaturated hydrocarbons can be made in all sorts of variants while still remaining within the scope of the invention.

## Claims

1. Method for preventing fouling due to condensation reactions of one or more carbonyls present or formed in an unsaturated hydrocarbon, characterized in that an additive formed by a solution of at least one hydride in a solvent is added to the unsaturated hydrocarbon.

2. Method according to the preceding claim, characterized in that this solvent is an alkali hydroxide solution.

3. Method according to any of the preceding claims, characterized in that the concentration of the hydride in the solvent amounts to 1 to 25 % by weight.

4. Method according to any of the preceding claims, characterized in that the hydride is a boro or aluminium hydride.

5. Method according to the preceding claim, characterized in that the hydride is an alkali borohydride or alkali aluminium hydride.

6. Method according to any of the preceding claims, characterized in that an amount of additive is added which amounts to between 0.0010 and 10 % by weight, dosed on the amount of hydrocarbon.

7. Method according to any of the preceding claims, characterized in that also an amount of promoter is added.

8. Method according to the preceding claim, characterized in that the promoter is added mixed with the hydride in the additive.

9. Method according to any of claims 7 and 8, characterized in that a substance is added as promoter from the group consisting of: $Pd(NO_3)_2$, $AlCl_3$, $CoCl_2$, $NiCl_2$, thiols, acetic acid, $TiCl_4$, diethyleneglycoldimethylether, glycolic acid, gallic acid, thiourea or mixtures thereof.

10. Method according to any of claims 7 to 9, characterized in that an amount of promoter is added which amounts to between 0.0010 and 10 % by weight, dosed on the amount of hydrocarbons.

11. Method according to any of the preceding claims, characterized in that the additive is added before or during a washing or extraction process to which the hydrocarbon is subjected.

12. Method according to the preceding claim, characterized in that the additive is added to the cracking gases supplied to the alkaline washing tower of an ethylene production installation.

## Patentansprüche

1. Verfahren zur Verhinderung von Faulprozessen aufgrund von Kondensationsreaktionen von einem oder mehreren Carbonylen, die in einem ungesättigten Kohlenwasserstoff vorhanden sind oder gebildet werden, dadurch gekennzeichnet, daß ein Additiv, gebildet durch eine Lösung von zumindest einem Hydrid in einem Lösungsmittel, dem ungesättigten Kohlenwasserstoff zugesetzt wird.

2. Verfahren gemäß dem vorgenannten Anspruch, dadurch gekennzeichnet, daß dieses Lösungsmittel eine Alkali-Hydroxidlösung ist.

3. Verfahren gemäß einem der vorgenannten Ansprüche, dadurch gekennzeichnet, daß die Konzentration des Hydrids im Lösungsmittel 1 bis 25 Gewichtsprozent beträgt.

4. Verfahren gemäß einem der vorgenannten Ansprüche, dadurch gekennzeichnet, daß das Hydrid ein Bor- oder Aluminiumhydrid ist.

5. Verfahren gemäß dem vorgenannten Anspruch, dadurch gekennzeichnet, daß das Hydrid ein Alkali-Borhydrid oder Alkali-Aluminiumhydrid ist.

6. Verfahren gemäß einem der vorgenannten Ansprüche, dadurch gekennzeichnet, daß eine Additivmenge zugesetzt wird, die sich auf zwischen 0,0010 und 10 Gewichtsprozent beläuft, dosiert auf die Kohlenwasserstoffmenge.

7. Verfahren gemäß einem der vorgenannten Ansprüche, dadurch gekennzeichnet, daß auch eine Quantität eines Aktivators zugesetzt wird.

8. Verfahren gemäß dem vorgenannten Anspruch, dadurch gekennzeichnet, daß der Aktivator mit dem Hydrid im Additiv vermischt zugesetzt wird.

9. Verfahren gemäß einem der Ansprüche 7 und 8, dadurch gekennzeichnet, daß als Aktivator eine Substanz zugesetzt wird aus der Gruppe, bestehend aus: $Pd(NO_3)_2$, $AlCl_3$, $CoCl_2$, $NiCl_2$, Thiole, Essigsäure, $TiCl_4$, Diethylenglykoldimethylether, Glykolsäure, Galisäure, Thioharnstoff oder Mischungen hiervon.

10. Verfahren gemäß einem der Ansprüche 7 bis 9, dadurch gekennzeichnet, daß eine Quantität eines Aktivators zugesetzt wird, die sich auf zwischen 0,0010 und 10 Gewichtsprozent beläuft, dosiert auf die Kohlenwasserstoffmenge.

**11.** Verfahren gemäß einem der vorgenannten Ansprüche, dadurch gekennzeichnet, daß das Additiv vor oder während eines Wasch- oder Extraktionsprozesses, dem der Kohlenwasserstoff unterworfen wird, zugesetzt wird.

**12.** Verfahren gemäß dem vorgenannten Anspruch, dadurch gekennzeichnet, daß das Additiv den Crackgasen zugesetzt wird, die dem Alkali-Waschturm einer Ethylenproduktionsvorrichtung zugeführt werden.

**Revendications**

**1.** Procédé pour empêcher l'encrassement dû à des réactions de condensation d'un ou de plusieurs groupements carbonyle présents ou formés dans un hydrocarbure insaturé, caractérisé en ce qu'on ajoute à l'hydrocarbure insaturé un additif formé par une solution d'au moins un hydrure dans un solvant.

**2.** Procédé selon la revendication précédente, caractérisé en ce que ce solvant est une solution d'hydroxyde de métal alcalin.

**3.** Procédé selon l'une quelconque des revendications précédentes, caractérisé en ce que la concentration de l'hydrure dans le solvant s'élève de 1 à 25% en poids.

**4.** Procédé selon l'une quelconque des revendications précédentes, caractérisé en ce que l'hydrure est un borohydrure ou un hydrure d'aluminium.

**5.** Procédé selon la revendication précédente, caractérisé en ce que l'hydrure est un borohydrure alcalin ou un hydrure d'aluminium alcalin.

**6.** Procédé selon l'une quelconque des revendications précédentes, caractérisé an ce qu'on ajoute une quantité d'additif qui s'élève à une valeur entre 0,0010 et 10% en poids, dosée sur la quantité de l'hydrocarbure.

**7.** Procédé selon l'une quelconque des revendications précédentes, caractérisé an ce qu'on ajoute également une quantité d'un promoteur.

**8.** Procédé selon la revendication précédente, caractérisé en ce qu'on ajoute le promoteur en mélange avec l'hydrure dans l'additif.

**9.** Procédé selon l'une quelconque des revendications 7 et 8, caractérisé en ce qu'on ajoute une substance comme promoteur choisie parmi le groupe constitué par: $Pd(NO_3)_2$, $AlCl_3$, $CoCl_2$, $NiCl_2$, des thiols, l'acide acétique, $TiCl_4$, l'éther diméthylique de diéthylèneglycol, l'acide glycolique, l'acide gallique, la thio-urée ou leurs mélanges.

**10.** Procédé selon l'une quelconque des revendications 7 à 9, caractérisé en ce qu'on ajoute une quantité de promoteur qui s'élève à une valeur entre 0,0010 et 10% en poids, dosée sur la quantité des hydrocarbures.

**11.** Procédé selon l'une quelconque des revendications précédentes, caractérisé en ce qu'on ajoute l'additif avant ou pendant un procédé de lavage ou d'extraction auquel on soumet l'hydrocarbure.

**12.** Procédé selon la revendication précédente, caractérisé en ce qu'on ajoute l'additif aux gaz de craquage qui alimentent la tour de lavage alcalin d'une installation de production d'éthylène.